# EUROPEAN PATENT SPECIFICATION

(11) **EP 3 104 874 B1**
(45) Date of publication and mention of the grant of the patent: **01.01.2020**
(21) Application number: 15704275.5
(22) Date of filing: 05.02.2015
(51) Int. Cl.: A61K 38/19, A61K 31/665, A61P 17/02, A61K 9/00, A61K 45/06, A61K 47/06, A61K 9/06, A61K 31/045, A61K 31/07, A61K 31/427, A61K 31/7036, A61K 31/7048, A61K 31/7056, A61K 47/64

(54) **COMPOSITIONS TO PROMOTE THE HEALING OF SKIN ULCERS AND WOUNDS**
ZUSAMMENSETZUNGEN ZUR FÖRDERUNG DER HEILUNG VON HAUTGESCHWÜREN UND -WUNDEN
COMPOSITIONS FAVORISANT LA CICATRISATION DES PLAIES ET ULCÈRES DE LA PEAU

(30) Priority: 05.02.2014 DK 201470059
(43) Date of publication of application: 21.12.2016
(73) Proprietor: Reponex Pharmaceuticals A/S, 2970 Hørsholm (DK)
(72) Inventor: HESLET, Lars, DK-2820 Gentofte (DK); UTTENTHAL, Lars Otto, E-28009 Madrid (ES)
(74) Representative: Zacco Denmark A/S
(86) International application number: PCT/EP2015/052411
(87) International publication number: WO 2015/118069

(56) References cited:
- EP-A1- 2 476 420
- EP-A2- 0 470 431
- WO-A1-92/14480
- DEL GIUDICE P ET AL: "Pseudomonas aeruginosa ecthyma gangrenosum and facial cellulitis complicating carbimazole-induced agranulocytosis", ARCHIVES OF DERMATOLOGY, AMERICAN MEDICAL ASSOCIATION, US, vol. 142, no. 12, December 2006 (2006-12), pages 1663-1664, XP009183673, ISSN: 0003-987X
- XINLEI HU ET AL: "Topically applied rhGM-CSF for the wound healing: A systematic review", BURNS, BUTTERWORTH HEINEMANN, GB, vol. 37, no. 5, 30 August 2010 (2010-08-30), pages 728-740, XP028238536, ISSN: 0305-4179, DOI: 10.1016/J.BURNS.2010.08.016 [retrieved on 2010-09-07]
- EVANS A V ET AL: "Recalcitrant ulcers in necrobiosis lipoidica diabeticorum healed by topical granulocyte-macrophage colony-stimulating factor", BRITISH JOURNAL OF DERMATOLOGY, OXFORD : WILEY-BLACKWELL, UK, vol. 147, no. 5, November 2002 (2002-11), pages 1023-1025, XP009183677, ISSN: 0007-0963, DOI: 10.1046/J.1365-2133.2002.49735.X
- HONG YAN ET AL: "Recombinant human granulocyte-macrophage colony-stimulating factor hydrogel promotes healing of deep partial thickness burn wounds", BURNS, BUTTERWORTH HEINEMANN, GB, vol. 38, no. 6, 2 February 2012 (2012-02-02), pages 877-881, XP028432190, ISSN: 0305-4179, DOI: 10.1016/J.BURNS.2012.02.001 [retrieved on 2012-02-14]
- STAGNO F ET AL: "Successful healing of hydroxyurea-related leg ulcers with topical granulocyte-macrophage colony-stimulating factor", BLOOD, AMERICAN SOCIETY OF HEMATOLOGY, US, vol. 94, no. 4, 15 August 1999 (1999-08-15), pages 1479-1480, XP009183676, ISSN: 0006-4971
- DATABASE WPI Week 200124 Thomson Scientific, London, GB; AN 2001-227133 XP002738639, & CN 1 273 824 A (UNIV SHENYANG PHARMACY) 22 November 2000 (2000-11-22)
- CHOI J-W: "Conjugation strategies for therapeutic proteins", INNOVATIONS IN PHARMACEUTICAL TECHNOLOGY, SAMEDAN LTD, GB, no. 24, December 2007 (2007-12), pages 42-46, XP009183712, ISSN: 1471-7204
- LISA POLLARO ET AL: "Strategies to prolong the plasma residence time of peptide drugs", MEDCHEMCOMM, ROYAL SOCIETY OF CHEMISTRY, UNITED KINGDOM , vol. 1, no. 5 December 2010 (2010-12), pages 319-324, XP002632706, ISSN: 2040-2503, DOI: 10.1039/C0MD00111B Retrieved from the Internet: URL:http://pubs.rsc.org/en/content/article pdf/2010/md/c0md00111b?page=search [retrieved on 2010-10-21]
- EL MAGHRABY GAMAL M M ET AL: "Can drug-bearing liposomes penetrate intact skin?", JOURNAL OF PHARMACY AND PHARMACOLOGY, JOHN WILEY & SONS LTD, LONDON; GB, vol. 58, no. 4, April 2006 (2006-04), pages 415-429, XP009158054, ISSN: 0022-3573, DOI: 10.1211/JPP.58.4.0001 [retrieved on 2010-02-18]

### SEQUENCE LISTING

<110> Reponex ApS
   Compositions to promote the healing of skin ulcers and wounds
<130> P81400330DK00
<160> 2
<170> PatentIn version 3.5
<210> 1
   <211> 144
   <212> PRT
   <213> Homo Sapiens
<400> 1
   Met Trp Leu Gln Ser Leu Leu Leu Leu Gly Thr Val Ala Cys Ser Ile
   1               5                   10                  15
   Ser Ala Pro Ala Arg Ser Pro Ser Pro Ser Thr Gln Pro Trp Glu His
               20                  25                  30
   Val Asn Ala Ile Gln Glu Ala Arg Arg Leu Leu Asn Leu Ser Arg Asp
           35                  40                  45
   Thr Ala Ala Glu Met Asn Glu Thr Val Glu Val Ile Ser Glu Met Phe
       50                  55                  60
   Asp Leu Gln Glu Pro Thr Cys Leu Gln Thr Arg Leu Glu Leu Tyr Lys
   65                  70                  75                  80
   Gln Gly Leu Arg Gly Ser Leu Thr Lys Leu Lys Gly Pro Leu Thr Met
                   85                  90                  95
   Met Ala Ser His Tyr Lys Gln His Cys Pro Pro Thr Pro Glu Thr Ser
               100                 105                 110
   Cys Ala Thr Gln Ile Ile Thr Phe Glu Ser Phe Lys Glu Asn Leu Lys
           115                 120                 125
   Asp Phe Leu Leu Val Ile Pro Phe Asp Cys Trp Glu Pro Val Gln Glu
       130                 135                 140
<210> 2
   <211> 144
   <212> PRT
   <213> Homo Sapiens
<400> 2

## Description

### Field of invention

The present invention provides compositions comprising as their essential ingredients granulocyte-macrophage colony-stimulating factor (GM-CSF) and the antibiotic fosfomycin, for the treatment of wounds, ulcers, sores, burns and other injuries of the skin or membranes of the body. As such, it is relevant to the fields of dermatology and nursing care in the areas of medicine and surgery, traumatology and burns.

### Background of the invention

### Wound healing

Wound healing is a normal feature of living animals and is a dynamic, interactive process which involves various types of cell and the extracellular matrix, depending for its speed and efficiency on various internal and external factors. The normal healing process can be described in terms of four programmed phases comprising 1) hemostasis, 2) inflammation, 3) proliferation and 4) remodeling.
1) Hemostasis is the vascular response stage that occurs immediately after the insult and normally lasts for up to a few hours. The wound may bleed initially, but blood clotting and vasoconstriction re-establish hemostasis while coagulated blood provides a provisional extracellular matrix for cell migration.
2) Inflammation normally starts at the time of injury and finishes within 3-4 days in the course of normal healing. The inflammation phase is clinically recognized by the classical signs and symptoms of inflammation, such as heat, redness, swelling and pain. The wound starts to exude fluid, which serves to remove debris, and proteases are released into the wound area. White blood cells and macrophages begin to congregate in the lesion to clear debris: Growth factors released by the macrophages stimulate fibroblasts. During this phase the extracellular matrix is constructed. Inflammation is always present, while the entry of bacteria may pass from colonization to clinical infection to exacerbate any delay in the resolution of the inflammatory phase.
3) Proliferation normally continues after the inflammatory phase and is characterized by the proliferation and migration of fibroblasts and the production of connective tissue. It starts about 4 days after the occurrence of the trauma and continues for 2-3 weeks in a normally healing closed wound. This phase can be extended significantly in an open wound with severe tissue damage, where complete closure will require the production of a large amount of connective tissue. During this phase, neovascularization, epithelialization, wound contraction and collagen production are taking place.
4) Remodeling (also called maturation, moderation or scar phase) starts 2-3 weeks after wound closure, while it does not start in open wounds before the wound has healed. It typically continues for several weeks, months or even years thereafter. Maturation involves contraction of the wound, growth of new epithelial tissue covering the granulation tissue, and possibly scar formation. During this phase myofibroblasts develop from the fibroblasts and the collagen fibers gradually mature and become relatively more organized.

For a wound to heal optimally, all four phases must occur in the proper sequence and timeframe. Different parts of a wound may heal at different rates, so that some parts of a normal wound may be at a more advanced stage of healing than others.

### Delayed wound healing and chronic or non-healing wounds and ulcers

Many factors may cause abnormal or impaired wound healing including prolonged failure to heal (non-healing wounds). Such wounds have generally failed to progress through the normal stages of healing and often enter a state of pathologic inflammation due to the delayed, incomplete, or uncoordinated healing process. These wounds are typically colonized or infected by one or more species of bacteria which exacerbate and prolong the inflammation and contribute to the delay in healing.

Most chronic wounds are ulcers that are associated with ischemia, diabetes mellitus, venous stasis or pressure. Non-healing wounds affect more than 1% of the industrialized world's population. In the United States they may affect up to 6 million people, about 85% of whom will be aged with persons 65 years or and older. Non-healing wounds result in enormous health care expenditures, with a total annual cost estimated at more than $3 billion in the United States.

The prevalence is expected to increase as the population ages and the number of individuals with diabetes mellitus increases. Chronic ulcers reduce the quality of life and working capacity of the patient and represent a substantial financial burden to the health care system.

Ulcers of the lower extremities, especially those attributed to diabetes mellitus, or venous or arterial insufficiency, comprise a substantial proportion of chronic ulcers. Approximately 15% to 25% of individuals with diabetes mellitus develop a foot ulcer at some point in their lifetime and an estimated 12% of those patients require lower extremity amputation. Healing is complicated by diabetic neuropathy and susceptibility to infection is greatly increased. Venous disease accounts for the majority of chronic lower extremity ulcers. Venous hypertension secondary to various causes can damage the blood vessel walls and ultimately leads to skin breakdown. Arterial ulcers are less common and are a result of impaired circulation which can adversely affect healing and lead to ulceration.

### Role of bacterial colonization and infection in chronic ulcers

In the inflammation phase of impaired wound healing, bacteria will always contaminate the ulcer in a process passing through colonization and critical colonization to infection. Critical colonization is not always associated with overt signs of infection but can result in failure to heal, poor-quality granulation tissue, increased wound friability, and increased exudation (Frank C et al., 2005). The microflora of chronic wounds such as ulcers most commonly exist in the biofilm phenotype and have been known to significantly impair normal healing trajectories (Smith DM et al., 2010). Standard treatment include the use of topical antimicrobials (iodine and silver preparations) or topical antibiotics (Mupirocin, fusidic acid, aminoglycosides, bacitracin, polymyxin B, gramicidins and metronidazole, alone or in various combinations) for two weeks or so. This may be followed by giving relevant oral antibiotics.

### Types of bacteria involved in chronic skin ulcers

The types of bacteria involved in colonization and infection of chronic skin ulcers typically include those that are present in the contralateral or surrounding intact skin, but will show an greatly enhanced presence of certain bacterial genera, including, for example, *Corynebacterium, Pseudomonas, Streptococcus, Escherichia, Shigella* and *Serratia* in a study of diabetic ulcers (Gontcharova V et al., 2010). Anaerobic genera such as *Finegoldia* and *Peptoniphilus* are also common. While *Staphylococcus* was not overrepresented as a genus, infection of chronic wounds with *S. aureus* must be taken seriously. In an analysis of decubitus ulcers, the most common bacterial genera identified were *Streptococcus, Corynebacterium, Staphylococcus, Finegoldia, Anaerococcus, Pseudomonas* and *Peptoniphilus.* Many functionally equivalent pathogroups (symbiotic colonies of otherwise nonpathogenic species that act synergistically to promote their own survival at the expense of the host), were found to colonize and exist as a chronic wound pathogenic biofilm, which is accepted to be the primary infectious agent in chronic wounds (Smith DM et al., 2010).

### Standard treatment of non-healing wounds and ulcers

Standard treatment for all non-healing wounds and ulcers includes debridement of necrotic tissue, infection control and local wound care. For each category of non-healing wound, specialized treatment modalities have to be implemented. For diabetic foot ulcers, for example, attention has to be paid to mechanical off-loading, tight blood glucose control and patient education on foot care. For venous ulcers, special measures typically include mechanical compression and limb elevation to reverse tissue edema and improve venous blood flow. Care for ulcers caused by arterial insufficiency is centered on reestablishing blood flow and minimizing further loss of tissue perfusion. For pressure ulcers, off-loading methods or devices are the gold standard of treatment.

If ulcers do not adequately heal with standard treatment, additional treatment modalities may be required, which are often termed "advanced wound care therapies." A large and growing array of advanced wound care therapies of different composition and indications have been developed. For a considerable number of these therapies their efficacy, comparative effectiveness and adverse effects are not well established. Thus, although there has been extensive research in the field of wound healing treatment, the healing of wounds and ulcers is still a complex task, particularly in the elderly or diseased population.

### Granulocyte-macrophage colony-stimulating factor (GM-CSF)

Granulocyte-macrophage colony stimulating factor (GM-CSF) is a member of the family of colony-stimulating factors (CSFs), which are glycoproteins that stimulate the proliferation and maturation of hematopoietic progenitors and enhance the functional activity of mature effector cells. In brief, at the level of the immature cells, CSF's ensure the self-renewal of the staminal pool and activate the first stage of hematopoietic differentiation. In the subsequent stage, when cell proliferation is associated with a progressive acquisition of the characteristics of the mature cells, they enormously enhance the number of differentiating cells. In the terminal stage, they stimulate the circulation and the activation of mature cells.

GM-CSF has been shown to have a positive effect on wound healing by facilitating wound contraction, causing local recruitment of inflammatory cells, and inducing keratinocyte proliferation. GM-CSF also activates mononuclear phagocytes, promotes migration of epithelial cells, and further regulates cytokine production in the healing process.

Mature GM-CSF is a monomeric protein of 127 amino-acid residues with several potential glycosylation sites. The variable degree of glycosylation results in a molecular weight range between 14 kDa and 35 kDa. Non-glycosylated and glycosylated GM-CSF show similar activity *in vitro* (Cebon J et al., 1990). The crystallographic analysis of GM-CSF revealed a barrel-shaped structure composed of four short alpha helices (Diederichs K et al., 1991). There are two known sequence variants of GM-CSF. The active form of the GM-CSF protein is found extracellularly as a homodimer *in vivo.*

GM-CSF exerts its biological activity by binding to its receptor. The most important sites of GM-CSF receptor (GM-CSF-R) expression are on the cell surface of myeloid cells, such as macrophages types I and II, epithelial cells and endothelial cells, whereas lymphocytes are GM-CSF-R negative. The native receptor is composed of alpha and beta subunits. The alpha subunit imparts ligand specificity and binds GM-CSF with nanomolar affinity. The beta subunit is also part of the interleukin-3 and interleukin-5 receptor complexes and, in association with the GM-CSF-R alpha subunit and GM-CSF, leads to the formation of a complex with picomolar binding affinity (Hayashida K et al., 1990). The binding domains on GM-CSF for the receptor have been mapped: GM-CSF interacts with the beta subunit of its receptor via a very restricted region in the first alpha helix of GM-CSF (Shanafelt AB et al., 1991a;b; Lopez AF et al., 1991). Binding to the alpha subunit could be mapped to the third alpha helix, helix C, the initial residues of the loop joining helices C and D, and to the carboxyterminal tail of GM-CSF (Brown CB et al., 1994).

Formation of the GM-CSF trimeric receptor complex leads to the activation of complex signaling cascades involving molecules of the JAK/STAT families, She, Ras, Raf, the MAP kinases, phosphatidylinositol-3 -kinase and NFkB, finally leading to the transcription of c-myc, c-fos and c-jun. Activation is mainly induced by the beta subunit of the receptor (Hayashida K et al., 1990; Kitamura T et al., 1991; Sato N et al., 1993). The shared beta subunit is also responsible for the overlapping functions exerted by IL-3, IL-5 and GM-CSF (reviewed by de Groot RP et al., 1998).

In addition to its stimulating activity on hemopoietic growth and differentiation, GM-CSF acts as a proinflammatory cytokine. Macrophages, e.g. macrophages type I & II and monocytes, as well as neutrophils and eosinophils, are activated by GM-CSF, resulting in the release of other cytokines and chemokines and matrix-degrading proteases, as well as increased expression of HLA and cell adhesion molecules or receptors for CC-chemokines. This in turn leads to increased chemotaxis of inflammatory cells into inflamed tissue.

Macrophages in wounds are known to release a variety of biologically active substances that serve as chemo-attractants for both monocytes and fibroblasts, such as transforming growth factor-β (TGF-β) and platelet-derived growth factor (PDGF). Activated macrophages digest devitalized collagen and the fibrin clot. Dissolution of the clot allows the formation of granulation tissue in the wound site during the second phase of wound healing.

### Use of GM-CSF to stimulate wound healing

The activities of GM-CSF outlined above not only stimulate the cellular proliferation, maturation and activities that are involved in the inflammation and proliferation stages of wound healing, but also, by the same processes, enhance the innate defense mechanisms against bacterial infection. The use of topically applied recombinant human GM-CSF to accelerate the healing of various types of chronic wounds and ulcers has been systematically reviewed and concluded to be beneficial for deep partial-thickness burns, chronic leg ulcers and leprosy ulcers, with some evidence for a positive effect on pressure ulcers and cancer-related ulcers (Hu X et al., 2011). Topically applied GM-CSF to experimental wounds infected with *Escherichia coli* in rats was found to stimulate the innate defense against infection as seen by reduced bacterial counts in association with accelerated wound closure (Robson M et al., 1994).

### The use of topical antibiotics to control bacterial infection in chronic wounds and ulcers

The current use of topical microbial agents and antibiotics in the standard care of chronic wounds and ulcers has been outlined above. Topical antimicrobials such as preparations based on iodine or silver may be likened to antiseptics in general: while they may have powerful bactericidal action, they also exert significant toxic effects on the living cells that are essential for the process of wound healing. The balance between the local bactericidal effect and the toxicity to the host tissue is an important consideration. This balance may be more favorable for the promotion of healing in the case of the antibiotics mentioned, but these antibiotics may also have significant cytotoxic actions at the local concentrations reached. It is evidently advantageous to choose an antibiotic or combination of antibiotics that is strongly bactericidal while having little or no toxicity towards the cells involved in wound healing. A further consideration is that the antibiotic should be active against the principal bacterial species that are known to occur in infected wounds and ulcers. In addition, it is a great advantage if the antibiotic is currently active against many multi-drug resistant bacterial strains that have become more prevalent in recent years. Finally, it is an advantage if there has been no appreciable increase in bacterial resistance to the drug over an extended period of time, so that there is a reasonable expectation that bacterial resistance to it will be

### Fosfomycin

Fosfomycin is the international non-proprietary name of a broad-spectrum antibiotic isolated and characterized in 1969 from *Streptomyces fradiae* strains under the name phosphomycin or phosphonomycin (Hendlin D et al., 1969). Its structure was determined to be (-)(IR, 2S)-1,2-epoxypropylphosphonic acid (Christensen BG et al., 1969), with the systematic (IUPAC) name [(2R,3S)-3-methyloxiran-2-yl]phosphonic acid and a formula weight of 138.1 Da. Fosfomycin is bactericidal and inhibits bacterial cell wall biosynthesis by inactivating the enzyme UDP-N-acetylglucosamine-3-enolpyruvyltransferase, also known as MurA (Brown ED et al., 1995). This enzyme catalyzes the committed step in peptidoglycan biosynthesis, the ligation of phosphoenolpyruvate to the 3'-hydroxyl group of UDP-N-acetylglucosamine to form N-acetylmuramic acid. Fosfomycin is a phosphoenolpyruvate analogue that inhibits MurA by alkylating an active site cysteine residue. The antibiotic enters the bacterial cell via the glycerophosphate transporter.

Given this mechanism of action, fosfomycin has a broad bactericidal spectrum, being active against aerobic genera such as *Staphylococcus, Streptococcus, Neisseria, Escherichia, Proteus* (indole-negative), *Serratia, Salmonella, Shigella, Pseudomonas, Haemophilus,* and *Vibrio,* less active against indole-positive *Proteus* spp., *Klebsiella* and *Enterobacter* spp. It is known to be active against the anaerobic genera *Peptostreptococcus* (including *Peptoniphilus, Finegoldia* and *Anaerococcus*) and *Fusobacterium.* It will be seen that this spectrum of activity comprises a large number of the genera prominent in critical colonization and infections of chronic skin ulcers.

There is a low prevalence of bacterial resistance to fosfomycin in the community, and studies of the prevalence of resistant bacteria after the introduction of fosfomycin have shown either no increase or only a modest increase in the prevalence of resistant organisms. However, prolonged exposure to the antibiotic may enable bacteria to evolve resistance by selection of mutants that lack the glycerophosphate transporter pathway. Alternative mechanisms of resistance involve the loss of the inducible hexose phosphate transporter, a Cys-Asp mutation in MurAS, or acquistion of plasmids coding for the fosfomycin inactivating enzymes fosA and fosB (in addition to the chromosomal fosX in *Listeria monocytogenes*). The mutant strains may, however, also show reduced pathogenicity (Karageorgopoulos DE et al., 2012). This may explain why the emergence of bacterial resistance is seen on prolonged exposure *in vitro,* but much less frequently *in vivo.* The appearance of resistant bacterial strains in controlled clinical trials of orally or intravenously administered fosfomycin has been 3.0% overall, with a maximum of 15% for *Pseudomonas aeruginosa.* In general, fosfomycin is seen to be a valuable addition to the therapeutic armament against multidrug-resistant organisms. There is no experience with the development of resistance to fosfomycin during treatment of bacterially infected chronic ulcers.

Fosfomycin has proved to be remarkably non-toxic to mammalian cells and organs, despite fosfomycin disodium being used at intravenous doses of up to 0.5 g/kg/day in human patients. Here the limiting factor is overload with the counter-ion rather than any toxic effect of the antibiotic. Indeed, fosfomycin has been found to exert a protective effect against the toxic action of other antibiotics, immunosuppressive or chemotherapeutic agents such as aminoglycosides, vancomycin, amphotericin B, polymyxin, ciclosporin and cisplatin (Gobernado M, 2003). As additional effects it has the capacity to favor phagocytosis and act as an immunomodulator. It is accumulated by polymorphonuclear leukocytes to reach concentrations that are twice those of the extracellular fluid, but does not affect their cellular functions, while exerting a bactericidal effect on *Staphylococcus aureus.* The chief adverse effects are gastric irritation from orally administered fosfomycin disodium, evidence of allergy in the form of transient rashes (0.3% of cases) and eosinophilia (0.2%), and transiently raised liver enzymes (0.3% of cases) (Gobernado M, 2003).

### The use of fosfomycin in combination with other antibiotics

Fosfomycin shows a considerable synergism in bactericidal effect on a large number of strains of organisms from the susceptible genera mentioned, when used in combination with a large number of antibiotics of the penicillin, cephalosporin, aminoglycoside, macrolide and lincosamide types. While early studies showed a synergistic effect on about 70-100% of tested strains for various antibiotic combinations, subsequent more extensive studies showed synergy rates of 36-74%. The remaining strains showed merely additive effects and an inhibitory effect was only seen in one or two individual antibiotic combinations on an individual bacterial strain (Gobernado M, 2003). The fact that fosfomycin shows synergy with many individual antibiotics and indeed abrogates the toxicity of many other antibiotics, including the nephrotoxicity and ototoxicity of the aminoglycosides, favors the use of fosfomycin in combination with other antibiotics to produce a potent bactericidal action and compensate for any development of fosfomycin resistance during more prolonged treatment.

### Summary of the invention

Accordingly, the present invention provides pharmaceutical compositions as defined in claim 1.

Secondly, a pharmaceutical composition according to the above, comprising one or more additional antibiotic or antimicrobial agents.

The advantage of combining GM-CSF with fosfomycin or fosfomycin in combination with other antibiotics is to exploit the known effect of GM-CSF to promote wound healing and the innate defense mechanisms against bacterial infection while further accelerating wound healing by more rapidly eliminating the infecting bacteria through the bactericidal action of fosfomycin, which has no toxic action on the granulocytes, macrophages and other cells through which the healing effect of GM-CSF is exerted. The effect is to achieve a faster rate of healing of wounds and ulcers showing delayed healing or non-healing than that achieved by existing treatments or by the use of either topical GM-CSF or topical fosfomycin, with or without an additional antibiotic, alone.

In the following detailed description of the invention, details of the scope of the invention and the meaning of the terms used will be given, together with details of the practical performance of the invention.

### Detailed description of the invention

The present invention provides compositions as defined in claim 1, and in a further embodiment, an additional antibiotic or antimicrobial agent, and in a still further embodiment, an additional agent which has been found to promote wound healing. Such compositions are useful for the treatment of wounds, ulcers, sores and other types of injury to the skin and mucous membranes and are intended to be topically applied in various ways which be further described. The compositions and methods of the present invention are also in some embodiments useful in methods of treatment.

### GM-CSF preparations

For practical purposes, the GM-CSF preparations to be used in the present invention will not be purified native human GM-CSF, which could of course be used if it were available in sufficient quantity and problems of possible viral contamination were overcome, but human GM-CSF prepared *in vitro* by recombinant DNA technology. The preparation of human recombinant GM-CSF (hrGM-CSF) in mammalian cells has been described (Wong GG et al., 1985; Kaushansky K et al., 1986). Similar work has led to the production of hrGM-CSF with the non-proprietary name regramostim in Chinese hamster ovarian (CHO) cells (first reported by Moonen P et al., 1987). The expression of hrGM-CSF in *Saccharomyces cerevisiae* was reported by Cantrell MA et al. (1985), leading to the preparation known by the non-proprietary name sargramostim. Sargramostim differs from endogenous human GM-CSF in having a leucine residue instead of a proline residue at position 23 of the pro-peptide and is less glycosylated than either endogenous human GM-CSF or regramostim (Armitage JO, 1998). The expression of hrGM-CSF in *Escherichia coli was* reported by Burgess AW et al. (1987), leading to the preparation known by the non-proprietary name molgramostim, which is not glycosylated. The hrGM-CSF preparations, sargramostim and molgramostim can be used in the present invention.

A "functional homologue" of human GM-CSF is herein defined as a polypeptide having at least 50% sequence identity with the known and naturally occurring sequence and sequence variants of human GM-CSF and has one or more functions of the naturally occurring protein. These functions include the following: stimulating the growth and differentiation of hematopoietic precursor cells from various lineages, including granulocytes, macrophages and monocytes, enhancing functional activities of mature effector cells involved in antigen presentation and cell-mediated immunity, including neutrophils, monocytes, macrophages, and dendritic cells. The functions also include those of particular relevance to wound healing, such as facilitating wound contraction, causing local recruitment of inflammatory cells, improving the recruitment of neutrophils, inducing keratinocyte proliferation, activating mononuclear phagocytes, promoting the migration of epithelial cells, and further regulating cytokine production in the healing process. Regramostim, sargramostim and molgramostim may all be said to be functional homologues of naturally occurring human GM-CSF.

Evolutionary conservation between GM-CSF homologues of different closely related species, as assessed by amino-acid sequence alignment, can be used to pinpoint the degree of evolutionary pressure on individual amino-acid residues. Preferably, GM-CSF sequences are compared between species where GM-CSF function is conserved, for example, but not limited to mammals, including rodents, monkeys and apes. Residues under high selective pressure are more likely to represent essential amino acid residues that cannot easily be substituted than residues that change between species. It is evident from the above that a reasonable number of modifications or alterations of the human GM-CSF sequence can be made without interfering with the activity of the GM-CSF molecule according to the invention. Such GM-CSF molecules are herein referred to as functional homologues of human GM-CSF, and may be such variants and fragments of native human GM-CSF as described below.

As used herein, the expression "variant" refers to a polypeptide or protein which is homologous to the index protein, which is naturally occurring human GM-CSF in the present instance, but which differs from the index protein in that one or more amino-acid residues within the sequence of the index protein are substituted by other amino-acid residues. These substitutions may be regarded as "conservative" when an amino-acid residue is replaced by a different amino-acid residue with broadly similar properties, and "non-conservative" when an amino-acid residue is replaced by one of a different type. Broadly speaking, fewer non-conservative substitutions will be possible without altering the biological activity of the polypeptide.

A person skilled in the art will know how to make and assess "conservative" amino-acid substitutions, by which one amino-acid residue is substituted by another having one or more shared chemical and/or physical characteristics. Conservative amino-acid substitutions are less likely to affect the functionality of the protein. Amino acids may be grouped according to their shared characteristics. A conservative amino-acid substitution is a substitution of one amino acid within a predetermined group of amino acids for another amino acid within the same group, within which the amino acids exhibit similar or substantially similar characteristics. Within the meaning of the term "conservative amino acid substitution" as applied herein, one amino acid may be substituted by another within groups of amino acids characterized by having
i) polar side chains (Asp, Glu, Lys, Arg, His, Asn, Gin, Ser, Thr, Tyr and Cys)
ii) non-polar side chains (Gly, Ala, Val, Leu, Ile, Phe, Trp, Pro and Met)
iii) aliphatic side chains (Gly, Ala Val, Leu and Ile)
iv) cyclic side chains (Phe, Tyr, Trp, His and Pro)
v) aromatic side chains (Phe, Tyr and Trp)
vi) acidic side chains (Asp and Glu)
vii) basic side chains (Lys, Arg and His)
viii) amide side chains (Asn and Gin)
ix) hydroxyl side chains (Ser and Thr)
x) sulfur-containing side chains (Cys and Met)
xi) amino acids being monoamino-dicarboxylic acids or monoaminomonocarboxylic-monoamidocarboxylic acids (Asp, Glu, Asn and Gin).

A functional homologue within the scope of the present invention is a polypeptide that exhibits at least 50% sequence identity with a naturally occurring form of human GM-CSF, such as at least 60% sequence identity, for example at least 70% sequence identity, such as at least 75% sequence identity, for example at least 80% sequence identity, such as at least 85 % sequence identity, for example at least 90 % sequence identity, such as at least 91 % sequence identity, for example at least 91% sequence identity, such as at least 92 % sequence identity, for example at least 93 % sequence identity, such as at least 94 % sequence identity, for example at least 95 % sequence identity, such as at least 96 % sequence identity, for example at least 97% sequence identity, such as at least 98 % sequence identity, for example 99% sequence identity with a naturally occurring form of human GM-CSF.

Sequence identity can be calculated using a number of well-known algorithms and applying a number of different gap penalties. Any sequence alignment algorithm, such as but not limited to FASTA, BLAST, or GETSEQ, may be used for searching homologues and calculating sequence identity. Moreover, when appropriate, any commonly known substitution matrix, such as but not limited to PAM, BLOSSUM or PSSM matrices, may be applied with the search algorithm. For example, a PSSM (position specific scoring matrix) may be applied via the PSI-BLAST program. Moreover, sequence alignments may be performed using a range of penalties for gap-opening and extension. For example, the BLAST algorithm may be used with a gap-opening penalty in the range 5-12, and a gap-extension penalty in the range 1-2.

Accordingly, a variant or a fragment thereof may comprise, within the same variant of the sequence or fragments thereof, or among different variants of the sequence or fragments thereof, at least one substitution, such as a plurality of substitutions introduced independently of one another.

It is clear from the above outline that the same variant or fragment thereof may comprise more than one conservative amino-acid substitution from more than one group of conservative amino acids as defined herein above.

Aside from the twenty standard amino acids and two special amino acids, selenocysteine and pyrrolysine, there are a vast number of "non-standard amino acids" which are not incorporated into protein *in vivo.* Examples of nonstandard amino acids include the sulfur-containing taurine and the neurotransmitters GABA and dopamine. Other examples are lanthionine, 2-aminoisobutyric acid, and dehydroalanine. Further non-standard amino are ornithine and citrulline.

Non-standard amino acids are usually formed through modifications to standard amino acids. For example, taurine can be formed by the decarboxylation of cysteine, while dopamine is synthesized from tyrosine and hydroxyproline is made by a posttranslational modification of proline (common in collagen). Examples of non-natural amino acids are those listed e.g. in 37 C.F.R. section 1.822(b)(4), all of which are referenced herein.

Both standard and non-standard amino acid residues described herein can be in the "D" or "L" isomeric form.

It is contemplated that a functional equivalent according to the invention may comprise any amino acid including non-standard amino acids. In preferred embodiments, a functional equivalent comprises only standard amino acids.

The standard and/or non-standard amino acids may be linked by peptide bonds or by non-peptide bonds. The term peptide also embraces post-translational modifications introduced by chemical or enzyme-catalyzed reactions, as are known in the art. Such post-translational modifications can be introduced prior to partitioning, if desired. Amino acids as specified herein will preferentially be in the L-stereoisomeric form. Amino acid analogs can be employed instead of the 20 naturally occurring amino acids. Several such analogs are known, including fluorophenylalanine, norleucine, azetidine-2-carboxylic acid, S-aminoethyl cysteine, 4-methyl tryptophan and the like.

In one embodiment of the present invention, the GM-CSF variant comprises a conjugate capable of prolonging half-life of the active ingredient, such as for example albumin or a fatty acid.

Suitable variants will be at least 60% identical, preferably at least 70%, and accordingly, variants preferably have at least 75% sequence identity, for example at least 80% sequence identity, such as at least 85 % sequence identity, for example at least 90 % sequence identity, such as at least 91 % sequence identity, for example at least 91% sequence identity, such as at least 92 % sequence identity, for example at least 93 % sequence identity, such as at least 94 % sequence identity, for example at least 95 % sequence identity, such as at least 96 % sequence identity, for example at least 97% sequence identity, such as at least 98 % sequence identity, for example 99% sequence identity with the predetermined sequence of a naturally occurring form of human GM-CSF.

Functional homologues may further comprise chemical modifications such as ubiquitination, labeling (e.g., with radionuclides, various enzymes, etc.), pegylation (derivatization with polyethylene glycol), or by insertion (or substitution by chemical synthesis) of amino acids such as ornithine, which do not normally occur in human proteins.

In addition to the peptidyl compounds described herein, sterically similar compounds may be formulated to mimic the key portions of the peptide structure and such compounds may also be used in the same manner as the polypeptides of the invention. This may be achieved by techniques of modelling and chemical designing known to those of skill in the art. For example, esterification and other alkylations may be employed to modify the amino terminus (N-terminus) of, e.g., a di-arginine peptide backbone, to mimic a tetrapeptide structure. It will be understood that all such sterically similar constructs fall within the scope of the present invention.

Peptides with N-terminal alkylations and C-terminal esterifications are also encompassed by the present invention. Functional equivalents also comprise glycosylated and covalent or aggregative conjugates formed with the same molecules, including dimers or unrelated chemical moieties. Such functional equivalents are prepared by linkage of functionalities to groups which are found in a fragment that includes any one or both of the N- and C-termini, by means known in the art.

The term "fragment thereof" may refer to any portion of the given amino-acid sequence. Fragments may comprise more than one portion from within the full-length protein, joined together. Suitable fragments may be deletion or addition mutants. The addition of at least one amino acid may be an addition of from preferably 2 to 250 amino acids, such as from 10 to 20 amino acids, for example from 20 to 30 amino acids, such as from 40 to 50 amino acids. Fragments may include small regions from the protein or combinations of these. The deletion and/or the addition may, independently of one another, be a deletion and/or an addition within a sequence and/or at the end of a sequence.

Deletion mutants suitably comprise at least 20 or 40 consecutive amino acid and more preferably at least 80 or 100 consecutive amino acids in length. Accordingly, such a fragment may be a shorter sequence taken from the sequence of human GM-CSF comprising at least 20 consecutive amino acids, for example at least 30 consecutive amino acids, such as at least 40 consecutive amino acids, for example at least 50 consecutive amino acids, such as at least 60 consecutive amino acids, for example at least 70 consecutive amino acids, such as at least 80 consecutive amino acids, for example at least 90 consecutive amino acids, such as at least 95 consecutive amino acids, such as at least 100 consecutive amino acids, such as at least 105 amino acids, for example at least 110 consecutive amino acids, such as at least 115 consecutive amino acids, for example at least 120 consecutive amino acids, wherein said deletion mutants preferably has at least 75% sequence identity, for example at least 80% sequence identity, such as at least 85 % sequence identity, for example at least 90 % sequence identity, such as at least 91 % sequence identity, for example at least 91% sequence identity, such as at least 92 % sequence identity, for example at least 93 % sequence identity, such as at least 94 % sequence identity, for example at least 95 % sequence identity, such as at least 96 % sequence identity, for example at least 97% sequence identity, such as at least 98 % sequence identity, for example 99% sequence identity with a naturally occurring form of human GM-CSF.

It is preferred that functional homologues of GM-CSF comprise at most 500, more preferably at most 400, even more preferably at most 300, yet more preferably at most 200, such as at most 175, for example at most 160, such as at most 150 amino acids, for example at most 144 amino acids.

There are two known variants of human GM-CSF: a T115I substitution in variant 1 and a I117T substitution in variant 2. Accordingly, in one embodiment of the invention, a functional homologue of GM-CSF comprises a sequence with high sequence identity to human GM-CSF NO: 1 or any of the splice variants.

Analogues of GM-CSF are, for example, described in U.S. Pat. Nos. 5,229,496, 5,393,870, and 5,391,485.

GM-CSF can be used in homo- or heteromeric form. Homo- and heteromeric forms of GM-CSF may comprise one or more GM-CSF monomers or functional homologous of GM-CSF as defined herein above. Homo- and heteromers include dimers, trimers, tetramers, pentamers, hexamers, heptamers, octamers, nonamers and decamers.

In one embodiment, a homodimer, trimer or tetramer of GM-CSF is used.

The amino-acid sequence of the precursor (including the signal peptide) form of GM-CSF of Homo sapiens (SEQ ID NO:1) is:

The amino-acid sequence of the corresponding mature protein (SEQ ID NO: 2) is:

Functional homologues of a naturally occurring form of human GM-CSF according to the present invention may be commercially available, e.g. sargramostim (Leukine®; Immunex, Seattle, WA, USA).

### Recombinant production of GM-CSF

GM-CSF or functional variants or homologues thereof can be produced in various ways, such as isolation from for example human or animal serum or from expression in cells, such as prokaryotic cells, yeast cells, insect cells, mammalian cells or in cell-free systems.

In one embodiment of the invention, GM-CSF is produced recombinantly by host cells. Thus, in one aspect of the present invention, GM-CSF is produced by host cells comprising a first nucleic acid sequence encoding the GM-CSF operably associated with a second nucleic acid sequence capable of directing expression in said host cells. The second nucleic acid sequence may thus comprise or even consist of a promoter that will direct the expression of protein of interest in said cells. A skilled person will be readily capable of identifying useful second nucleic acid sequence for use in a given host cell.

The process of producing a recombinant GM-CSF in general comprises the steps of
- providing a host cell
- preparing a gene expression construct comprising a first nucleic acid sequence encoding the GM-CSF operably linked to a second nucleic acid sequence capable of directing the expression of said protein of interest in the host cell
- transforming the host cell with the construct
- cultivating the host cell, thereby obtaining expression of the GM-CSF.

The recombinant GM-CSF thus produced may be isolated by any conventional method, such as any of the methods for protein isolation described herein below. The skilled person will be able to identify suitable protein isolation steps for purifying the GM-CSF.

In one embodiment of the invention, the recombinantly produced GM-CSF is excreted by the host cells. When the GM-CSF is excreted, the process of producing a recombinant protein of interest may comprise the steps of
- providing a host cell
- preparing a gene expression construct comprising a first nucleic acid sequence encoding the GM-CSF operably linked to a second nucleic acid sequence capable of directing the expression of said protein of interest in said host cell
- transforming said host cell with the construct
- cultivating the host cell, thereby obtaining expression of the GM-CSF and secretion of the GM-CSF into the culture medium
- thereby obtaining culture medium containing the GM-CSF.

The composition comprising GM-CSF and nucleic acids may thus in this embodiment of the invention be the culture medium or a composition prepared from the culture medium.

In another embodiment of the invention, said composition is an extract prepared from animals, parts thereof or cells or an isolated fraction of such an extract.

In an embodiment of the invention, the GM-CSF is recombinantly produced *in vitro* in host cells and isolated from cell lysate, cell extract or from tissue culture supernatant. In a more preferred embodiment, the GM-CSF is produced by host cells that are modified in such a way that they express the relevant GM-CSF. In an even more preferred embodiment of the invention, said host cells are transformed to produce and excrete the relevant GM-CSF.

Pharmaceutical compositions according to the present invention may comprise GM-CSF or functional variants or homologues thereof at a concentration of 1 µg/mL to 10 mg/mL, more preferably 5 µg/mL to 500 µg/mL, and even more preferably 10 µg/mL to 200 µg/mL.

### Fosfomycin preparations

The fosfomycin preparations that fall within the scope of the present invention are salts of compounds that comprise the structure (-)(IR, 2S)-1,2-epoxypropylphosphonic acid, systematic name [(2R,3S)-3-methyloxiran-2-yl]phosphonic acid, formula weight 138.1 Da. Although the free acid form of this structure is used as a basis for calculating the effective amounts and concentrations of fosfomycin, the free acid is unstable and the antibiotic is presented for clinical use as a calcium salt. The principal form of fosfomycin in current use that come within the scope of this invention are:
i) Fosfomycin calcium monohydrate, formula weight 194.1 Da, pH of 0.4% solution 8.1-9.6. This salt is sparingly soluble in water but is less irritating to the stomach and is used for oral treatment. Its bioavailability may be as low as 12% (Bergan T, 1990).

Common to fosfomycin preparations is that they are stable for at least 3 years as dry powders at 25°C, but show a pH-dependent instability in aqueous solution, at 25°C losing 10% of fosfomycin activity within 10 h at pH 3, 2 months at pH 6.5 and 2 years at pH 9.75. Reduced stability at low pH must be taken into in account when formulating aqueous compositions for topical application with a suitable shelf life.

### Combination with other antimicrobial agents

A composition of the present invention may also contain one or more additional antimicrobial agents to potentiate its bactericidal action on important pathogens such as *Staphylococcus aureus* or *Pseudomonas aeruginosa,* prevent the overgrowth of any strains that develop resistance on prolonged treatment, or broaden the antimicrobial spectrum to include non-bacterial pathogens, including but not limited to fungi. Non-limiting examples of antimicrobial agents that may be included in the composition are: fusidic acid or sodium fusidate, penicillins, cephalosporins, aminoglycosides, macrolides, vancomycin, lincomycin, clindamycin, fluoroquinolones, mupirocin, bacitracin, polymyxin B, gramicidins, metronidazole, clotrimazole, ketoconazole and nystatin. Particularly suitable for inclusion are agents which show a synergic bactericidal action with fosfomycin, non-limiting examples of which are: penicillin, ampicillin, carbenicillin, methicillin, oxacillin, mezlocillin, piperacillin, aztreonam, imipenem, cephalexin, cephalothin, cefamandole, cefoxitin, cefmetazole, cefotaxime, cefazolin, cefoperazone, cefsulodine, ceftadizime, cefepime, streptomycin, gentamicin, kanamycin, netilmicin, tobramycin, amikacin, erythromycin, midecamycin, vancomycin, lincomycin, clindamaycin, teicoplanin, daptomycin, ciprofloxacin, ofloxazine, levofloxazin, pefloxacin, sparfloxacin. Certain antibiotics are incompatible with fosfomycin in aqueous solution, such as ampicillin, methampicillin, cephaloridine, cephalothin, streptomycin, gentamicin, kanamycin, a feature that may make them unsuitable for aqueous compositions within the scope of this invention.

In one embodiment, the additional antibiotic or antibacterial agent is selected from the list of arbekacin, aztreonam, cefoxitin, cefoperazone, cephalexin, clindamycin, flucloxacillin, meropenem, metronidazole, mezlocillin, or vancomycin. The selection of clindamycin or meropenem or metronidazole is particularly intended for the treatment of wounds suspected or known through appropriate bacterial culture to be infected with anaerobic bacteria including *Bacteroides* species.

In another embodiment, the additional antibiotic is at least a combination of aztreonam and arbekacin. This is particularly intended for the treatment of wounds suspected or known through appropriate bacterial culture to be infected with *Pseudomonas aeruginosa.*

As all the above compounds are known to the skilled person, it will not present an undue burden to the skilled person to choose from the list of compounds which compound(s) to combine with the GM-CSF and fosfomycin treatment on the basis of test data on the identity of the infective organisms that are to be treated.

### Further ingredients of the pharmaceutical composition

Vitamin A and anti-oxidant agents may further have a stimulating effect on the tissue during the process of healing. In one embodiment of the present invention, the composition as defined herein further comprises vitamin A and/or an anti-oxidant agent, non-limiting examples of which are: vitamin E (in the form of alpha-tocopherol), ubiquinone, idebenone, carotenoids such as lycopene, ascorbic acid or ascorbates, and nicotinamide.

### Dose

By "effective amount" of the pharmaceutical compositions of the present invention is meant a dose, which, when administered to a subject in need thereof, achieves a concentration which has a beneficial biological effect, i.e. by treatment, prevention or alleviation of irritation or lesion such as wounds, ulcers and other lesions of the skin, mucosal membranes or connective tissue of the body. Such an effective amount may be determined by a patient's attending physician or veterinarian and is readily ascertained by one of ordinary skill in the art. Factors which influence what a therapeutically effective amount will be include the following:
i) The specific activity of the therapeutic agent being used,
ii) The type of lesion (mechanical or thermal, full or partial thickness, etc.)
iii) The size of the lesion
iv) The depth of the lesion (if full thickness)
v) The presence of infection and the type of infection
vi) The time elapsed since the infliction of the injury infliction
vii) The existence of other disease states
viii) The age, physical condition, and nutritional status of the patient
Other medication that the patient may be receiving will affect the determination of the therapeutically effective amount of the therapeutic agent to administer.

Whereas the effective amounts and dosages of the ingredients of the a pharmaceutical composition are determined in relation to body weight or body surface area for systemic treatments, for topical application of a composition to wounds and ulcers, the effective amounts and dosages are more appropriately expressed in terms of the area of wound or ulcer to be treated, e.g. expressed in square centimeters, provided that the systemic absorption of the active ingredients does not lead to an adversely high dosage for the patient.

The effective amount of GM-CSF or a functional variant or homologue thereof, for topical application to a wound or ulcer may be from 1 microgram (µg) to 100 µg per square centimeter per day, such as in the range of 2 µg to 80 µg per square centimeter per day, and especially in the range of 5 µg to 50 µg per square centimeter per day.

The effective amount is expressed in terms of the amount to be given of a fully functional homologue of human GM-CSF such as molgramostim and is adjusted according to functional activity of the homologue used.

In practical terms, a pharmaceutical composition of the present invention comprises GM-CSF or a fragment or variant thereof at a concentration in the range of 1 µg/mL (or µg/g) to 10 mg/mL (or mg/g), such as in the range of 5 µg/mL (or µg/g) to 500 µg/mL (or µg/g), or such as in the range of 10 µg/mL (or µg/g) to 200 µg/mL (or µg/g).

The effective amount of a suitable salt of fosfomycin for topical application to a wound or ulcer may be from 10 mg to 1000 µg per square centimeter per day, such as in the range of 20 µg to 800 µg per square centimeter per day, and especially in the range of 50 µg to 500 µg per square centimeter per day. The effective amount is expressed in terms of the content of fosfomycin free acid in the preparation used.

In practical terms, a pharmaceutical composition of the present invention comprises a fosfomycin salt at a concentration in the range of 100 µg/mL (or µg/g) to 30 mg/mL (or mg/mL), such as in the range of 250 µg/mL (or µg/g) to 10 mg/mL (or mg/g) in terms of fosfomycin free acid.

The effective amount of an additional antimicrobial agent for topical application is known in the art and may be added to the pharmaceutical compositions of the present invention in an amount that is adjusted so that when an effective amount of GM-CSF and fosfomycin is given, an effective amount of the additional antimicrobial agent is also given. In practice, this means that fusidic acid, for example, will be added to the composition in a similar amount to that of fosfomycin, while tobramycin, for example, will be added in an amount that is between 1% and 10% of the amount of fosfomycin.

The effective daily dose is preferably administered once a day, but may be administered in divided doses twice a day, three times a day, four times a day, five times a day or six times a day.

Duration of dosing will typically range from 1 day to about 4 months, such as in the range of 1 day to 2 days, 2 days to 3 days, 3 days to 4 days, 4 days to 5 days, 5 days to 6 days, or in the range of 1 week to 2 weeks, 2 weeks to 3 weeks, 3 weeks to 4 weeks, or in the range of 1 month to 2 months, 2 months to 3 months, 3 months to 4 months, as long as the lesion remains unhealed.

The transformation of a resting macrophage into a fully immunocompetent dendritic cell after *in vitro* incubation of macrophages with GM-CSF takes approximately 10 days. In one embodiment, a duration of a dose has the length allowing for said a transformation, thus the duration can be in the range of 7 days to 14 days, such as 8 days to 12 days, for example 8 days, or 9 days, or 10 days, or 11 days, or 12 days.

A dose regime may alternate between periods of administration of the pharmaceutical composition according to the present invention and periods with no administration (a pause in treatment). A period with a pause of treatment in such a dose regime may last for 5 days to 10 days, for example 5 days, or 6 days, or 7 days, or 8 days, or 9 days, or for example 10 days or more, for example 1 to 4 months.

Examples of dosage regimes may include a cycle of 10 days of treatment with the pharmaceutical composition according to the present invention and 7 days' pause of treatment. The pause of treatment may be prolonged to 2 or 3 weeks or more, up to 6 weeks, if alternative treatment with a preparation that does not contain antibiotic is substituted. This is to prevent the generation of antibiotic-resistant organisms in the individual wound.

The conversion of resting macrophages into dendritic cells may be boosted by repeating a dosage regime. Thus dosage regimes can be repeated one, two, three, four, five or more times in order to obtain an effective treatment.

In one embodiment, a dosage regime is repeated, such as once, two times, three times or more times, for example repeated for the rest of the lifespan of a subject in need.

In another embodiment, patients are treated with a dosage regime of 10 days treatment with pharmaceutical composition according to the present invention, followed by a pause of 7-20 days in said treatment and subsequently repeating the dosage regime 2-3 or more times.

### Formulations

The pharmaceutical composition of the present invention is in the form of a powder. The active ingredients of the composition may be suspended as a micronized powder in non-aqueous media or dissolved in aqueous media.

A dusting powder is a preferred formulation, as the active ingredients of the composition are compatible with each other and show long-term stability at room temperature in the dry powder form. The formulation may contain powder additives such as starch stearate, cellulose, lactose, zinc oxide, silicon dioxide, magnesium carbonate, talc or clay.

**Example 2** gives the composition of an ointment to provide an effective dose of GM-CSF and fosfomycin in a single daily application.

**Example 3** gives the composition of an ointment to provide an effective dose of GM-CSF and fosfomycin together with clindamycin in a single daily application to provide a strong action against anaerobic bacteria and also against methicillin-resistant *Staphylococcus aureus* (MRSA).

**Example 4** gives the composition of an ointment to provide an effective dose of GM-CSF and fosfomycin together with arbekacin and aztreonam in a single daily application to provide a strong action against *Pseudomonas aeruginosa.*

The compositions according to the present invention may be formulated for improved penetration and efficacy of the active ingredients in their passage over the transmucosal barrier or epidermis. Improved passage over the transmucosal barrier may be obtained by a formulation which is capable of adhering to the mucosa, epidermis, or wound surface.

Formulations according to the present invention may comprise pharmaceutically acceptable carriers and excipients including microspheres, liposomes, micelles, microcapsules, nanoparticles or the like. The GM-CSF component may, for example, be formulated in a liposome with an outer fatty layer with a core of water phase in which the GM-CSF component is dissolved. The lipid layer of such formulations overcomes the penetration barrier of the epidermis or mucous membrane.

Conventional liposomes are typically composed of phospholipids (neutral or negatively charged) and/or cholesterol. The liposomes are vesicular structures based on lipid bilayers surrounding aqueous compartments. They can vary in their physicochemical properties such as size, lipid composition, surface charge and number and fluidity of the phospholipids bilayers. The most frequently used lipid for liposome formation are: 1,2-dilauroyl-*sn*-glycero-3-phosphocholine (DLPC), 1,2-dimyristoyl-*sn*-glycero-3-phosphocholine (DMPC), 1,2-dipalmitoyl-*sn*-glycero-3-phosphocholine (DPPC), 1,2-distearoyl-*sn*-glycero-3-phosphocholine (DSPC), 1,2-dioleoyl-*sn*-glycero-3-phosphocholine (DOPC), 1,2-dimyristoyl-*sn*-glycero-3-phosphoethanolamine (DMPE), 1,2-dipalmitoyl-*sn*-glycero-3-phosphoethanolamine (DPPE), 1,2-dioleoyl-*sn*-glycero-3-phosphoethanolamine (DOPE), 1,2-dimyristoyl-*sn*-glycero-3-phosphate (monosodium salt) (DMPA), 1,2-dipalmitoyl-*sn*-glycero-3-phosphate (monosodium salt) (DPPA), 1,2-dioleoyl-*sn*-glycero-3-phosphate (monosodium salt) (DOPA), 1,2-dimyristoyl-*sn-*glycero-3-[phospho-*rac*-(1-glycerol)] (sodium salt) (DMPG), 1,2-dipalmitoyl-*sn*-glycero-3-[phospho-*rac*-(1-glycerol)] (sodium salt) (DPPG), 1,2-dioleoyl-*sn*-glycero-3-[phospho*rac*-(1-glycerol)] (sodium salt) (DOPG), 1,2-dimyristoyl-*sn*-glycero-3-[phospho-l-serine] (sodium salt) (DMPS), 1,2-dipalmitoyl-*sn*-glycero-3-[phospho-l-serine) (sodium salt) (DPPS), 1,2-dioleoyl-*sn*-glycero-3-[phospho-l-serine] (sodium salt) (DOPS), 1,2-dioleoyl-*sn*-glycero-3-phosphoethanolamine-n-(glutaryl) (sodium salt) and 1,1',2,2'-tetramyristoyl cardiolipin (ammonium salt). Formulations composed of DPPC in combination with other lipids or modifiers of liposomes are preferred, e.g. in combination with cholesterol and/or phosphatidylcholine.

A useful way of producing liposomes is to attach hydrophilic polymer polyethylene glycol (PEG) covalently to the outer surface of the liposome. Some of the preferred lipids are: 1,2-dipalmitoyl-*sn*-glycero-3-phosphoethanolamine-n-[methoxy(polyethylene glycol)-2000] (ammonium salt), 1,2-dipalmitoyl-sn-glycero-3-phosphoethanolamine-n-[methoxy(polyethylene glycol)-5000] (ammonium salt), 1,2-dioleoyl-3-trimethylammonium-propane (chloride salt) (DOTAP).

Possible lipids applicable for liposomes are supplied by e.g. Avanti, Polar Lipids, Inc., Alabaster, AL, USA. Additionally, the liposome suspension may include lipid-protective agents which protect lipids against free-radical and lipid-peroxidative damage on storage. Lipophilic free-radical quenchers, such as alpha-tocopherol and water-soluble iron-specific chelators, such as ferrioxamine, are preferred.

Several methods are available for preparing liposomes, as described in, e.g., Szoka F et al. (1980), U.S. Pat. Nos. 4, 235,871, 4,501,728 and 4,837,028, all of which are referenced herein. Another method produces multi-lamellar vesicles of heterogeneous sizes. In this method, the vesicle-forming lipids are dissolved in a suitable organic solvent or solvent system and dried under vacuum or an inert gas to form a thin lipid film. If desired, the film may be re-dissolved in a suitable solvent, such as tertiary butanol, and then lyophilized to form a more homogeneous lipid mixture which is in a more easily hydrated powder-like form. This film is covered with an aqueous solution of the targeted drug and the targeting component and allowed to hydrate, typically over a 15-60 minute period with agitation. The size distribution of the resulting multi-lamellar vesicles can be shifted toward smaller sizes by hydrating the lipids under more vigorous agitation conditions or by adding solubilizing detergents such as deoxycholate.

To ensure adequate shelf-life of the components of the dermatological formulation, especially that of fosfomycin, which is unstable in aqueous media at physiological pH, while at the same time providing a formulation that is acceptable to the patient in terms of irritation at the site of application, an embodiment of the invention comprises the provision of the active components in a kit as separate parts to be mixed shortly before use. The kit may further comprise a medium into which the components can be mixed prior to the topical application to the wound. The medium may contain, in addition to other ingredients, buffering agents such as those mentioned above to ensure that the pH of the formulation is appropriate for dermatological use once all the ingredients have been mixed.

### Indications

The present invention provides pharmaceutical compositions for use in the treatment of wounds, ulcers, sores, burns or other lesions of the skin, mucosal membranes or connective tissue of the body, which may be acute or chronic. Such lesions may be caused by a broad spectrum of events and/or may be associated with other diseases. The lesions to be treated include those associated with incision, laceration, abrasion, blister, hematoma, puncture, penetration, gunshot, electricity, irradiation, chemical, trauma, crush, bite, burn, frost, surgery, primary cancer or metastasis, benign tumor, acne, infections such as bacterial infection (which may be combined with fungal or viral of parasitic infection), lesions associated with decreased circulation of blood, such as leg ulcers and foot ulcers associated with venous insufficiency or arterial insufficiency, decubitus ulcers, pressure sores or bedsores, and lesions associated with diabetes mellitus.

Chronic wounds, now generally called "non-healing wounds", or lesions, wounds or ulcers arise when a wound fails to follow an appropriate timely healing process to achieve the normal sustained and stable anatomic and functional integrity of the healed tissue. Generally speaking, a skin lesion which has failed to make substantial progress towards healing within a period of three months, or which has become stable in a partially healed state for more than three months, could be categorized as a chronic or "non-healing" wound. This general definition is not universally applicable, as the age and fitness of the patient, as well as other factors such as diseases or disorders suffered by the patient (for example, circulatory disorders), can significantly lengthen the normal healing process. In such circumstances a skin lesion which is unhealed after six months can be categorized as a "non-healing" wound.

A "non-healing" wound or chronic skin lesion is ulcerous when it involves focal loss of epidermis and at least part of the dermis. Chronic ulcerous skin lesions are usually accompanied by other symptoms apart from the failure of the normal healing process. Typical accompanying signs and symptoms include one or more of the following: pain, exudation, bad smell, excoriation, wound spreading, tissue necrosis, irritation and hyperkeratosis. Such symptoms can be extremely debilitating and embarrassing for patients, and can seriously harm the patient's quality of life. In severe cases, they can necessitate the amputation of limbs or even cause death.

Malignant or pre-malignant chronic ulcerous skin lesions may arise in connection with a primary cancer of the skin, or with a metastasis to the skin from a local tumor or from a tumor at a distant site. They may be draining or non-draining. They may, for example, take the form of a cavity, an open area on the surface of the skin, skin nodules, or a nodular growth extending from the surface of the skin.

The pharmaceutical compositions of the present invention are useful for treatment of all the above-mentioned non-healing wounds or chronic ulcerous skin lesions, and thus reduce or prevent one or more symptoms accompanying such lesions.

**Disclosed herein are:**
1. A pharmaceutical composition comprising
   a. granulocyte-macrophage colony-stimulating factor (GM-CSF) or a fragment or variant thereof, and
   b. fosfomycin in the form of an inorganic or organic salt thereof.
2. The pharmaceutical composition according to embodiment 1 comprising one or more additional antibiotic or antimicrobial agents.
3. The composition according to embodiment 2, wherein the one or more additional antibiotic or antimicrobial agent is selected from the list of fusidic acid, penicillins, cephalosporins, aminoglycosides, macrolides, vancomycin, lincomycin, clindamycin, fluoroquinolones, mupirocin, bacitracin, polymyxin B, gramicidins, metronidazole, clotrimazole, ketoconazole and nystatin.
4. The composition according to embodiment 2 or 3, wherein the one or more additional antibiotic or antimicrobial agent is selected from the list of penicillin, ampicillin, carbenicillin, methicillin, oxacillin, flucloxacillin, mezlocillin, piperacillin, aztreonam, imipenem, cephalexin, cephalothin, cefamandole, cefoxitin, cefmetazole, cefotaxime, cefazolin, cefoperazone, cefsulodine, ceftadizime, cefepime, streptomycin, gentamicin, kanamycin, netilmicin, tobramycin, amikacin, erythromycin, midecamycin, vancomycin, lincomycin, clindamaycin, teicoplanin, daptomycin, ciprofloxacin, ofloxazine, levofloxazin, pefloxacin, sparfloxacin, ceftriaxone, arbekacin, or vancomycin.
5. The composition according to any one of the preceding embodiments, wherein the wound or lesion is infected with staphylococcus such as *Staphylococcus aureus* or pseudomonas such as *Pseudomonas aeruginosa.*
6. The composition according to any one of the preceding embodiments, wherein the composition is not in an aqueous solution, and wherein the additional antibiotic or microbial agent is any one of ampicillin, methampicillin, cephaloridine, cephalothin, streptomycin, gentamicin, or kanamycin.
7. The composition of any one of the preceding embodiments, wherein the composition further comprises one or more of the following: vitamin A, an anti-oxidant agent, such as one or more of the following: vitamin E (in the form of alpha-tocopherol), ubiquinone, idebenone, carotenoids in example lycopene, ascorbic acid or ascorbates, or nicotinamide.
8. The pharmaceutical composition according to any one of the previous embodiments, wherein the composition is for the treatment, alleviation or accelerating the healing of a lesion such as a wound, ulcer, sore or burn of any one of the skin, mucosal membranes or connective tissue underlying the lesion.
9. The pharmaceutical composition according to anyone of the previous embodiments, wherein the lesion is chronic.
10. The pharmaceutical composition according to any one of embodiments 1-11, wherein the lesion is acute.
11. The pharmaceutical composition according to any one of the previous embodiments, wherein the lesion is associated with colonization or infection by a bacterium, fungus, virus, or parasite.
12. The composition according to any one of the previous embodiments, wherein the lesion is infected with bacteria from any one of the genera *Staphylococcus, Streptococcus, Neisseria, Escherichia, Proteus, Serratia, Salmonella, Shigella, Pseudomonas, Haemophilus,* and *Vibrio, Proteus* spp., *Klebsiella* and *Enterobacter* spp., *Peptostreptococcus* (including *Peptoniphilus, Finegoldia* and *Anaerococcus*) and *Fusobacterium.*
13. The composition according to any one of the previous embodiments, wherein the active components are provided in a kit as separate parts to be mixed shortly before use.
14. The kit according to embodiment 13, wherein the kit further comprises a medium into which the components can be mixed prior to the topical application to the wound.
15. The pharmaceutical composition according to any one of the previous embodiments, wherein the lesion is associated with diabetes mellitus.
16. The pharmaceutical composition according to any one of the previous embodiments, wherein the lesion is associated with decreased circulation of blood, such as venous leg ulcers, venous foot ulcers, arterial leg ulcers, arterial foot ulcers, and decubitus ulcers.
17. The pharmaceutical composition according to any one of the previous embodiments formulated for topical application as a powder, paste, ointment, lotion, gel, cream, salve, emulsion, suspension, solution, spray, sponge, strip, plaster, pad, dressing, or formulated in an ostomy plate.
18. The composition according to any one of the previous embodiments, wherein GM-CSF and fosfomycin is for topical administration, and the additional antimicrobial drug is for non-topical administration.
19. The pharmaceutical composition according to any one of embodiments 1 to 14 wherein the GM-CSF is in a liposomal or micelle or microcapsule or nanoparticle formulation.
20. The pharmaceutical composition according to any one of the preceding embodiments wherein the GM-CSF variant is at least 70% identical to SEQ ID NO:1 or 2.
21. The pharmaceutical composition according to any one of the preceding embodiments wherein the GM-CSF fragment comprises at least 50 contiguous amino acid residues of any one of SEQ ID NO:1 or 2.
22. The pharmaceutical composition according to embodiment 21, wherein the fragment is at least 70% identical to SEQ ID NO:1 or 2 in the range of overlap.
23. The pharmaceutical composition according to any one of the preceding embodiments, comprising GM-CSF or a fragment or variant thereof at a concentration of 1 µg/mL to 10 mg/mL.
24. The pharmaceutical composition according to any one of the preceding embodiments, comprising GM-CSF or a fragment or variant thereof at a concentration of 5 µg/mL to 500 µg/mL.
25. The pharmaceutical composition according to any one of the preceding embodiments, comprising GM-CSF or a fragment or variant thereof at a concentration of 10 µg/mL to 200 µg/mL.
26. The pharmaceutical composition according to any one of the preceding embodiments, comprising a fosfomycin salt at a concentration of 100 µg/mL to 10 mg/mL in terms of fosfomycin free acid.
27. The pharmaceutical composition according to any one of the preceding embodiments, comprising a fosfomycin salt at a concentration pf 250 µg/mL to 1 mg/mL in terms of fosfomycin free acid.
28. The pharmaceutical composition according to embodiments 17 and 18, wherein the fosfomycin salt is fosfomycin disodium or fosfomycin calcium or fosfomycin trometamol, also known as fosfomycin tromethamine.
29. The pharmaceutical composition according to any one of the preceding embodiments, further comprising one or more additional antibiotic or antimicrobial agents.
30. The pharmaceutical composition according to any one of the preceding embodiments further comprising vitamin A and/or an anti-oxidant agent.
31. The pharmaceutical composition according to any one of the preceding embodiments, wherein the GM-CSF variant comprises a conjugate capable of prolonging the half-life of the GM-CSF.
32. The pharmaceutical composition according to the preceding embodiment, wherein the conjugate capable of prolonging half-life of the GM-CSF variant is albumin or a fatty acid.
33. The pharmaceutical compositions according to any one of the preceeding claims are also usefull in methods of treatment.

### Examples

The following non-limiting examples further illustrate the present invention.

### Example 1: Sequences

**SEQ ID NO: 1** - **Human GM-CSF precursor**
   >sp|P04141|CSF2_HUMAN Granulocyte-macrophage colony-stimulating factor OS=Homo sapiens
**SEQ ID NO: 2 - mature human GM-CSF**
   >sp|P04141|18-144

### Example 2: Ointment for treating wounds, ulcers, sores or burns of the skin

| | | |
|---|---|---|
| Molgramostim micronized | 0.5 mg | 0.05% |
| Fosfomycin trometamol micronized | 5 mg | 0.5% |
| Chlorbutanol, anhydrous | 5 mg | 0.5% |
| Mineral oil | 50 mg | 5% |
| White Petrolatum | to 1 g | To 100% |

This composition may be especially suitable for leg ulcers due to venous insufficiency, which do not show clinical signs of gross bacterial infection.

### Example 3: Ointment for treating wounds, ulcers, sores or burns of the skin, which contains a further antibiotic in addition to fosfomycin (comparative)

| | | |
|---|---|---|
| Molgramostim micronized | 0.5 mg | 0.05% |
| Fosfomycin trometamol micronized | 5 mg | 0.5% |
| Clindamycin phosphate micronized | 10 mg | 1.0% |
| Chlorbutanol, anhydrous | 5 mg | 0.5% |
| Mineral oil | 50 mg | 5% |
| White Petrolatum | to 1 g | To 100% |

This composition may be especially suitable for treating wounds and ulcers in which infection with anaerobic bacteria is suspected or verified by anaerobic bacterial culture. It may also be suitable for wounds infected with methicillin-resistant *Staphylococcus aureus* (MRSA) verified by culture to be sensitive to clindamycin.

### Example 4: Ointment for treating wounds, ulcers, sores or burns of the skin, which contains a further two antibiotics in addition to fosfomycin (comparative)

| | | |
|---|---|---|
| Molgramostim micronized | 0.5 mg | 0.05% |
| Fosfomycin trometamol micronized | 5 mg | 0.5% |
| Arbekacin sulfate micronized | 2 mg | 0.2% |
| Aztreonam micronized | 5 mg | 0.5% |
| Chlorbutanol, anhydrous | 5 mg | 0.5% |
| Mineral oil | 50 mg | 5% |
| White Petrolatum | to 1 g | To 100% |

This composition may be especially suitable for treating wounds and ulcers in which infection with *Pseudomonas aeruginosa* is suspected or verified by bacterial culture.

### References

Armitage JO (1998) Emerging applications of recombinant human granulocyte-macrophage colony-stimulating factor. Blood 92:4491-4508.
Brown CB, Pihl CE, Kaushansky K. (1994) Mapping of human granulocyte-macrophage-colony-stimulating-factor domains interacting with the human granulocyte-macrophage-colony-stimulating-factor-receptor alpha-subunit. Eur J Biochem 225:873-880.
Brown ED, Vivas El, Walsh CT, Kolter R (1995) MurA (MurZ), the enzyme that catalyzes the first committed step in peptidoglycan biosynthesis, is essential in Escherichia coli. J Bacteriol 177:4194-4197.
Burgess AW, Begley CG, Johnson GR, Lopez AF, Williamson DJ, Mermod JJ, Simpson RJ, Schmitz A, DeLamarter JF (1987) Purification and properties of bacterially synthesized human granulocyte-macrophage colony stimulating factor. Blood 69:43-51.
Cantrell MA, Anderson D, Cerretti DP, Price V, McKereghan K, Tushinski RJ, Mochizuki DY, Larsen A, Grabstein K, Gillis S, et al (1985) Cloning, sequence, and expression of a human granulocyte/macrophage colony-stimulating factor. Proc Natl Acad Sci USA 82:6250-6254.
Cebon J, Nicola N, Ward M, Gardner I, Dempsey P, Layton J, Dührsen U, Burgess AW, Nice E, Morstyn G (1990) Granulocyte-macrophage colony stimulating factor from human lymphocytes. The effect of glycosylation on receptor binding and biological activity. J Biol Chem 265:4483-4491.
Christensen BG, Leanza WJ, Beattie TR, Patchett AA, Arison BH, Ormond RE, Kuehl FA Jr, Albers-Schonberg G, Jardetzky O (1969) Phosphonomycin: structure and synthesis. Science 166:123-125.
Diederichs K, Jacques S, Boone T, Karplus PA (1991) Low-resolution structure of recombinant human granulocyte-macrophage colony stimulating factor. J Mol Biol 221:55-60.
Frank C, Bayoumi I, Westendorp C (2005) Approach to infected skin ulcers. Can Fam Physician 51:1352-1359.
Gobernado M (2003) Fosfomycin. Rev Esp Quimioter 16:15-40.
Gontcharova V, Youn E, Sun Y, Wolcott RD, Dowd SE (2010) A comparison of bacterial composition in diabetic ulcers and contralateral intact skin. Open Microbiol J 4:8-19.
de Groot RP, Coffer PJ, Koenderman L (1998) Regulation of proliferation, differentiation and survival by the IL-3/IL-5/GM-CSF receptor family. Cell Signal 10:619-628.
Hayashida K, Kitamura T, Gorman DM, Arai K, Yokota T, Miyajima A (1990) Molecular cloning of a second subunit of the receptor for human granulocyte-macrophage colony-stimulating factor (GM-CSF): reconstitution of a high-affinity GM-CSF receptor. Proc Natl Acad Sci USA 87:9655-9659.
Hendlin D, Stapley EO, Jackson M, Wallick H, Miller AK, Wolf FJ, Miller TW, Chaiet L, Kahan FM, Foltz EL, Woodruff HB, Mata JM, Hernandez S, Mochales S (1969) Phosphonomycin, a new antibiotic produced by strains of streptomyces. Science 166: 122-123.
Hu X, Sun H, Han C, Wang X, Yu W (2011) Topically applied rhGM-CSF for the wound healing: a systematic review. Burns 37:729-741.
Karageorgopoulos DE, Wang R, Yu XH, Falagas ME (2012) Fosfomycin: evaluation of the published evidence on the emergence of antimicrobial resistance in Gram-negative pathogens. J Antimicrob Chemother 67:255-268.
Kaushansky K, O'Hara PJ, Berkner K, Segal GM, Hagen FS, Adamson JW (1986) Genomic cloning, characterization, and multilineage growth-promoting activity of human granulocyte-macrophage colony-stimulating factor. Proc Natl Acad Sci USA 83:3101-3105.
Kitamura T, Hayashida K, Sakamaki K, Yokota T, Arai K, Miyajima A. Reconstitution of functional receptors for human granulocyte/macrophage colony-stimulating factor (GM-CSF): evidence that the protein encoded by the AIC2B cDNA is a subunit of the murine GM-CSF receptor. Proc Natl Acad Sci USA 88:5082-5086.
Lopez AF, Vadas MA, Woodcock JM, Milton SE, Lewis A, Elliott MJ, Gillis D, Ireland R, Olwell E, Park LS. (1991) Interleukin-5, interleukin-3, and granulocyte-macrophage colony-stimulating factor cross-compete for binding to cell surface receptors on human eosinophils. J Biol Chem 266:24741-24747.
Moonen P, Mermod JJ, Ernst JF, Hirschi M, DeLamarter JF (1987) Increased biological activity of deglycosylated recombinant human granulocyte/macrophage colony-stimulating factor produced by yeast or animal cells. Proc Natl Acad Sci USA 84:4428-4431.
Robson M, Kucukcelebi A, Carp SS, Hayward PG, Hui PS, Cowan WT, Ko F, Cooper DM (1994) Effects of granulocyte-macrophage colony-stimulating factor on wound contraction. Eur J Clin Microbiol Infect Dis 3 Suppl 2:S41-S46.
Sato N, Sakamaki K, Terada N, Arai K, Miyajima A (1993) Signal transduction by the high-affinity GM-CSF receptor: two distinct cytoplasmic regions of the common beta subunit responsible for different signaling. EMBO J 12:4181-4189.
Shanafelt AB, Miyajima A, Kitamura T, Kastelein RA (1991a) The amino-terminal helix of GM-CSF and IL-5 governs high affinity binding to their receptors. EMBO J 10:4105-4112.
Shanafelt AB, Johnson KE, Kastelein RA (1991b) Identification of critical amino acid residues in human and mouse granulocyte-macrophage colony-stimulating factor and their involvement in species specificity. J Biol Chem 266:13804-13810.
Smith DM, Snow DE, Rees E, Zischkau AM, Hanson JD, Wolcott RD, Sun Y, White J, Kumar S, Dowd SE (2010) Evaluation of the bacterial diversity of pressure ulcers using bTEFAP pyrosequencing. BMC Med Genomics 3:41.
Szoka F Jr, Papahadjopoulos D (1980) Comparative properties and methods of preparation of lipid vesicles (liposomes). Annu Rev Biophys Bioeng 9:467-508.
Wong GG, Witek JS, Temple PA, Wilkens KM, Leary AC, Luxenberg DP, Jones SS, Brown EL, Kay RM, Orr EC, et al (1985) Human GM-CSF: molecular cloning of the complementary DNA and purification of the natural and recombinant proteins. Science 228:810-815.

## Claims

1. A pharmaceutical composition comprising
a. granulocyte-macrophage colony-stimulating factor (GM-CSF) in the form of molgramostim or sargramostim, and
b. fosfomycin calcium salt
for topical use in a method of treating, alleviating, or accelerating of the healing of, a lesion such as a wound, ulcer, sore or burn of the skin, mucosal membranes or connective tissue underlying the lesion, wherein said pharmaceutical composition is formulated as a powder.

2. The pharmaceutical composition for topical use according to claim 1, the composition comprising one or more additional antibiotic or antimicrobial agents.

3. The pharmaceutical composition for topical use according to any one of claims 1 to 2, wherein the lesion is colonized or infected by a bacterium, fungus, virus, or parasite.

4. The pharmaceutical composition for topical use according to any one of claims 1 to 3, wherein the lesion is in individuals with diabetes mellitus.

5. The pharmaceutical composition for topical use according to any one of the preceding claims, comprising GM-CSF at a concentration of 1 µg/g to 10 mg/g.

6. The pharmaceutical composition for topical use according to any one of the preceding claims, comprising GM-CSF at a concentration of 5 µg/g to 500 µg/g.

7. The pharmaceutical composition for topical use according to any one of the preceding claims, comprising GM-CSF at a concentration of 10 µg/g to 200 µg/g.

## Patentansprüche

1. Pharmazeutische Zusammensetzung umfassend
a. Granulocyt-Makrophagen-Kolonie-stimulierenden Faktor (GM-CSF) in Form von Molgramostim oder Sargramostim, und
b. Phosphomycin-Kalzium-Salz
zur topischen Anwendung in einem Verfahren zur Behandlung, Linderung oder Beschleunigung der Heilung einer Läsion, zum Beispiel einer Wunde, eines Geschwürs, einer offenen Stelle oder Verbrennung der Haut, Schleimhautmembranen oder des Bindegewebes unter der Läsion, wobei die pharmazeutische Zusammensetzung als ein Pulver formuliert ist.

2. Pharmazeutische Zusammensetzung zur topischen Anwendung nach Anspruch 1, wobei die Zusammensetzung ein oder mehrere zusätzliche Antiobiotika oder antimikrobielle Mittel umfasst.

3. Pharmazeutische Zusammensetzung zur topischen Anwendung nach einem der Ansprüche 1 bis 2, wobei die Läsion von einem Bakterium, Pilz, Virus oder Parasiten kolonisiert oder infiziert ist.

4. Pharmazeutische Zusammensetzung zur topischen Anwendung nach einem der Ansprüche 1 bis 3, wobei die Läsion Individuen mit Diabetes mellitus betrifft.

5. Pharmazeutische Zusammensetzung zur topischen Anwendung nach einem der vorhergehenden Ansprüche, umfassend GM-CSF in einer Konzentration von 1 µg/g bis 10 mg/g.

6. Pharmazeutische Zusammensetzung zur topischen Anwendung nach einem der vorhergehenden Ansprüche, umfassend GM-CSF in einer Konzentration von 5 µg/g bis 500 µg/g.

7. Pharmazeutische Zusammensetzung zur topischen Anwendung nach einem der vorhergehenden Ansprüche, umfassend GM-CSF in einer Konzentration von 10 µg/g bis 200 µg/g.

## Revendications

1. Composition pharmaceutique comprenant
a. un facteur de stimulation des colonies de granulocytes-macrophages (GM-CSF) sous la forme de molgramostim ou de sargramostim, et
b. un sel de calcium de fosfomycine
pour une application topique dans un procédé de traitement, de soulagement ou d'accélération de la cicatrisation d'une lésion, telle qu'une blessure, un ulcère, une plaie ou une brûlure de la peau, des membranes muqueuses ou du tissu conjonctif sous-jacent à la lésion, ladite composition pharmaceutique étant formulée en tant que poudre.

2. Composition pharmaceutique pour une application topique selon la revendication 1, la composition comprenant un ou plusieurs agents antibiotiques ou antimicrobiens supplémentaires.

3. Composition pharmaceutique pour une application topique selon l'une quelconque des revendications 1 à 2, dans laquelle la lésion est colonisée ou infectée par une bactérie, un champignon, un virus ou un parasite.

4. Composition pharmaceutique pour une application topique selon l'une quelconque des revendications 1 à 3, dans laquelle la lésion est présente dans des individus avec un diabète sucré.

5. Composition pharmaceutique pour une application topique selon l'une quelconque des revendications précédentes, comprenant GM-CSF à une concentration de 1 µg/g à 10 mg/g.

6. Composition pharmaceutique pour une application topique selon l'une quelconque des revendications précédentes, comprenant GM-CSF à une concentration de 5 µg/g à 500 µg/g.

7. Composition pharmaceutique pour une application topique selon l'une quelconque des revendications précédentes, comprenant GM-CSF à une concentration de 10 µg/g à 200 µg/g.
